# EUROPEAN PATENT SPECIFICATION

(11) **EP 2 968 632 B1**
(45) Date of publication and mention of the grant of the patent: **30.12.2020**
(21) Application number: 14770164.3
(22) Date of filing: 13.03.2014
(51) Int. Cl.: A61L 2/20, A61L 2/00

(54) **PROCESS FOR FOCUSED GAS PHASE APPLICATION OF BIOCIDE**
VERFAHREN FÜR FOKUSSIERTE GASPHASENANWENDUNG EINES BIOZIDS
PROCÉDÉ POUR L'APPLICATION CIBLÉE DE BIOCIDE EN PHASE GAZEUSE

(30) Priority: 15.03.2013 US 201313836721
(43) Date of publication of application: 20.01.2016
(73) Proprietor: Sabre Intellectual Property Holdings LLC, Slingerlands, NY 12159 (US)
(72) Inventor: MASON, John Y., Slingerlands NY 12159 (US)
(74) Representative: HGF
(86) International application number: PCT/US2014/025884
(87) International publication number: WO 2014/151512

(56) References cited:
- CN-U- 201 572 402
- GB-A- 2 396 559
- US-A- 1 443 315
- US-A1- 2003 136 426
- US-A1- 2004 062 680
- US-A1- 2007 163 935
- US-A1- 2009 071 474
- US-A1- 2010 310 418
- US-A1- 2010 310 418
- US-A1- 2011 238 004
- US-A1- 2011 262 342
- US-A1- 2013 164 385
- Heidi Hubbard ET AL: "Chlorine Dioxide Reactions with Indoor Materials during Building Disinfection: Surface Uptake", Environmental Science & Technology, vol. 43, no. 5, 1 March 2009 (2009-03-01), pages 1329-1335, XP055691693, US ISSN: 0013-936X, DOI: 10.1021/es801930c

## Description

The present invention relates to a process for focused gas phase application of biocide. The present invention generally relates to methods for oxidizing, sanitizing, disinfecting, and/or sterilizing a target. More particularly, the present invention relates to methods for oxidizing, sanitizing, disinfecting, and/or sterilizing a target utilizing focused gas phase application of chlorine dioxide.

### BACKGROUND OF THE INVENTION

The use of chlorine dioxide (ClO₂) as, e.g, an oxidizing, sanitizing, disinfecting, or sterilizing agent is known. Chlorine dioxide is a powerful biocide, the bactericidal, algicidal, fungicidal, bleaching, and deodorizing properties of which are well known. It has been employed in a wide spectrum of applications, including the disinfection of food (*see, e.g.,* Trinetta et. al., Food Microbiology 27 (2010) 1009-1015), odor control, wound treatment (*see, e.g.,* U.S. Patent No. 8,311,625), bleaching, microbial decontamination, mold remediation, Chinese wallboard remediation, and disinfection of medical waste.

Gas phase application of chlorine dioxide has been used, for example, to remediate targets in buildings contaminated with, e.g., bacteria, spores, molds, mycotoxins, allergens, insects, larvae, and/or arachnids (*see, e.g.,* U.S. Patent No. 8,192,684). It is generally accepted that in order to achieve adequate kill through such application, chlorine dioxide fumigation of a space requires a target chlorine dioxide concentration and exposure time of 750 ppmᵥ for 12 hours, for a total concentration of 9000 ppmv-hrs (CT). Under current EPA guidelines, applications of gaseous chlorine dioxide for building remediation require 75% relative humidity and an exposure of 9000 ppmv-hrs.

Despite the numerous applications of chlorine dioxide in various forms, various drawbacks to known methods of utilizing the biocide exist. For example, gas phase applications of chlorine dioxide are known in the art to require high concentration-time (CT) values to achieve desired levels of kill of targeted organisms.

Thus, a need exists for improved methods and devices for effective gas phase application of chlorine dioxide.

In this specification, where a document, act or item of knowledge is referred to or discussed, this reference or discussion is not an admission that the document, act or item of knowledge or any combination thereof was, at the priority date, publicly available, known to the public, part of common general knowledge, or otherwise constitutes prior art under the applicable statutory provisions; or is known to be relevant to an attempt to solve any problem with which this specification is concerned.

### SUMMARY OF THE INVENTION

Briefly, the present invention satisfies the need for improved methods and devices for effective gas phase application of chlorine dioxide. The present invention may address one or more of the problems and deficiencies of the art discussed above. However, it is contemplated that the invention may prove useful in addressing other problems and deficiencies in a number of technical areas. Therefore, the claimed invention should not necessarily be construed as limited to addressing any of the particular problems or deficiencies discussed herein.

In one aspect, the invention provides a method of oxidizing, sanitizing, disinfecting, and/or sterilizing a target. The method includes: ejecting a gas stream of a gaseous mixture comprising 50 to 30,000 ppmᵥ chlorine dioxide from a gas source at a velocity of 45.7 to 274.3 metres per second (m/s) (150 to 900 ft/sec); and contacting the gas stream with the target.

We also describe a device for oxidizing, sanitizing, disinfecting, and/or sterilizing a target. The device includes: a chlorine dioxide inlet configured for intake of a gaseous mixture comprising 50 to 30,000 ppmᵥ chlorine dioxide; and a gas source configured to eject a gas stream of the gaseous mixture at a velocity of 45.7 to 274.3 m/s (150 to 900 ft/sec).

Certain embodiments of the presently-disclosed methods and devices for oxidizing, sanitizing, disinfecting, and/or sterilizing a target have several features, no single one of which is solely responsible for their desirable attributes. Without limiting the scope of these methods as defined by the claims that follow, their more prominent features will now be discussed briefly. After considering this discussion, and particularly after reading the section of this specification entitled "Detailed Description of the Invention," one will understand how the features of the various embodiments disclosed herein provide a number of advantages over the current state of the art. These advantages may include, without limitation, providing improved methods for oxidizing, sanitizing, disinfecting, and/or sterilizing a target, providing methods for oxidizing, sanitizing, disinfecting, and/or sterilizing a target that are capable of utilizing focused gas phase application of chlorine dioxide, providing methods that work in enclosed and non-enclosed (open) spaces and application zones, providing improved methods for both large and small scale applications, and providing methods capable of oxidizing, sanitizing, disinfecting, and/or sterilizing a target at lower CT's than prior art methods and devices. Additionally, it is envisioned that the invention would allow for the focused application of chlorine dioxide for these purposes without exposing areas other than the target to chlorine dioxide or causing air exposure issues to chlorine dioxide gas to organisms including macrorganisms being treated or operating personnel.

These and other features and advantages of this invention will become apparent from the following detailed description of the various aspects of the invention taken in conjunction with the appended claims and the accompanying drawings.

### BRIEF DESCRIPTION OF THE DRAWINGS

FIG. 1 is a side perspective line drawing of a device for oxidizing, sanitizing, disinfecting, and/or sterilizing a target for implementing one embodiment of the process of the present invention.
FIG. 2 illustrates a device for oxidizing, sanitizing, disinfecting, and/or sterilizing a target for implementing an embodiment of the process of the present invention.
FIG. 3 illustrates a device for oxidizing, sanitizing, disinfecting, and/or sterilizing a target for implementing another embodiment of the process of the present invention.

### DETAILED DESCRIPTION OF THE INVENTION

The present invention is generally directed to methods for oxidizing, sanitizing, disinfecting, and/or sterilizing a target.

Although this invention is susceptible to embodiment in many different forms, certain embodiments of the invention are shown and described. It should be understood, however, that the present disclosure is to be considered as an exemplification of the principles of this invention and is not intended to limit the invention to the embodiments illustrated.

Reference numerals retain their designation and meaning for the same or like or similar elements throughout the various drawings.

In one aspect, the invention provides a method of oxidizing, sanitizing, disinfecting, and/or sterilizing a target. The method includes: ejecting a gas stream of a gaseous mixture comprising 50 to 30,000 ppmᵥ chlorine dioxide from a gas source at a velocity of 45.7-274.3 m/s (150 to 900 ft/sec); and contacting the gas stream with the target.

As used herein, "oxidizing" refers to the phenomenon of oxidation, which is the combination of a substance (e.g., a target) with oxygen, and/or a reaction in which the atoms in an element (e.g., of a target) lose electrons and the valence of the element is correspondingly increased.

As used herein, "sanitizing" refers to the phenomenon of sanitization, which is the process of making something (e.g., a target, such as an inanimate object) clean. Sanitization refers to a 3-log reduction.

As used herein, "disinfecting" refers to the phenomenon of disinfection, which is the process of eliminating pathogenic organisms on a target or making them inert, i.e., to kill or render harmless, e.g., germs and/or bacteria. Disinfection refers to a 4-log reduction.

As used herein, "sterilizing" refers to the phenomenon of sterilization, which is the process of completely eliminating microbial viability, e.g., to kill all non-pathogenic and pathogenic spores, fungi, bacteria, and viruses. Sterilization refers to a 6-log reduction (synonymous herein with "6-log kill"), which is the statistical destruction of all microorganisms and their spores. This is defined as 6 logs (10⁶) or a 99.9999% reduction. Statistically, this definition is accepted as zero viable organisms surviving.

As will be apparent to a person having ordinary skill in the art, generally speaking, sterilizing refers to a higher standard of kill than does disinfecting, than does sanitizing, than does oxidizing. Accordingly, methods of sterilizing according to the invention also comprise methods of oxidizing, sanitizing, and disinfecting. Further, generally speaking, in various embodiments, methods of disinfecting comprise methods of sanitizing and oxidizing; and methods of sanitizing also comprise methods of oxidizing. Since oxidizing does not refer to a specific level of kill, methods of oxidizing likewise may, but need not, comprise methods of sanitizing, disinfecting, and/or sterilizing.

In various embodiments of the invention, oxidizing, sanitizing, disinfecting, and/or sterilizing a target may comprise, for example: decontamination of a target; cleaning of a target, lightening or whitening a target; and/or restoratively treating a target.

As used herein, "target" refers to anything (e.g., cell(s), object, surface, structure, space, etc.) that a user may intend to subject to oxidation, sanitization, disinfection, and/or sterilization. In various embodiments, a target is in need of oxidation, sanitization, disinfection, and/or sterilization. The method of the invention are configured for oxidizing, sanitizing, disinfecting, or sterilizing, alone or in any combination, any desired target.

In some embodiments of the invention, the target is located in a large structure (e.g., a building) and/or high area application. In some embodiments of the invention, the target is such that application of the inventive method is through a focused, small scale application.

In various embodiments of the invention, the target intended to be oxidized, sanitized, disinfected, and/or sterilized may be, but is not limited to, one or more of: (a) a ceiling or wall, or a portion thereof; (b) a medical (e.g., general, surgical or dental) instrument, or a portion thereof; (c) an area of skin (e.g., hands or a portion thereof); (d) a wound or a portion thereof (e.g., a mammalian or human wound or portion thereof); (e) a medical procedural area, or a portion thereof; (f) a piece of artwork of a portion thereof; (g) any bacteria, spores, fungi, molds, mycotoxins, viruses allergens, insects, larvae, and/or arachnids; and (h) any other cell(s), object, surface, structure, space, etc. in need of, and/or comprising a contaminant in need of oxidation, sanitization, disinfection, and/or sterilization.

In certain embodiments, the invention provides oxidative methods for, e.g., lightening or whitening treatments (e.g., for teeth) or restorative treatments (e.g., of artwork).

In certain embodiments, the invention provides methods for, e.g., sterilizing target such as hands (e.g., a hand blower that oxidizes, sanitizes, disinfects and/or sterilizes hands,).

In certain embodiments of the invention (e.g., for treating wounds), the provided methods use a gaseous mixture that is substantially non-cytotoxic.

In some embodiments of the invention, decontaminating a target comprises oxidizing, sanitizing, disinfecting, and/or sterilizing a target. For example, various embodiments of the invention relate to decontaminating a target in a large structure (e.g., a building) and/or high area application.

Methods of the invention comprise ejecting a gas stream of a gaseous mixture. The gaseous mixture comprises 50 to 30,000 ppmᵥ chlorine dioxide. This concentration may also be referred to as the time-weighted average of chlorine dioxide concentration in parts per million by volume, meaning that for any time period "X" that the gaseous mixture is dispensed/ejected over, the recited concentration is the average concentration of gas dispensed/ejected over the period (e.g., if gas is ejected for a two minute period in a concentration of 100 ppmᵥ during the first minute, and 300 ppmᵥ during the second minute, the time-weighted average of chlorine dioxide concentration over the two-minute period would be 200 ppmᵥ).

For example, in some embodiments, the gaseous mixture comprises 50, 100, 500, 1,000, 1,500, 2,000, 2,500, 3,000, 3,500, 4,000, 4,500, 5,000, 5,500, 6,000, 6,500, 7,000, 7,500, 8,000, 8,500, 9,000, 9,500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 20,500, 21,000, 21,500, 22,000, 22,500, 23,000, 23,500, 24,000, 24,500, 25,000, 25,500, 26,000, 26,500, 27,000, 27,500, 28,000, 28,500, 29,000, 29,500, or 30,000 ppmᵥ chlorine dioxide, including any and all ranges and subranges therein (e.g., 1,000 to 15,000 ppmᵥ, 1,500 to 10,000 ppmᵥ, 2,000 to 10,000 ppmᵥ, 2,000 to 8,000 ppmᵥ, 2,500 to 3,500 ppmᵥ, etc.).

In some embodiments, the gaseous mixture further comprises nitrogen, oxygen, argon, and/or carbon dioxide. In a preferred embodiment, the gaseous mixture comprises chlorine dioxide and air. In some embodiments, the gaseous mixture consists essentially of chlorine dioxide and air.

In some embodiments of the invention (e.g., in methods for treating wounds), the gaseous mixture may comprise one or more additional therapeutic agents.

Methods of the invention comprise ejecting the gaseous mixture from a gas source. As used herein, the gas source may be any suitable mechanism that is capable of ejecting gas therefrom, whether alone (e.g., in the case of a tube with gas pumping therethrough), or together with one or more other components (e.g., in the case of a nozzle, which may require one or more additional components to allow for the ejection of gas therefrom, e.g., a motor or other device/component that causes gas to flow through the source). For example, in some non-limiting embodiments, the gas source may be a nozzle, spout, tap, valve, receptacle, pipe, tube, hose, fan, duct outlet, etc.

In one embodiment, the gas source is a nozzle. In some embodiments, the nozzle has a diameter of 0.05 to 1 cm.

The gas source of the invention comprises, or is configured such that it is attached/connected to (either directly or indirectly), a chlorine dioxide source. The chlorine dioxide source may be any suitable source comprising chlorine dioxide. For example, the gas source comprises, or is configured such that it is connected to a chlorine dioxide source that comprises, and optionally produces chlorine dioxide. Examples of such chlorine dioxide sources include, for example, a receptacle comprising chlorine dioxide gas or a solution comprising, e.g., dissolved chlorine dioxide. The chlorine dioxide gas or solution may have been produced by any acceptable means prior to introduction into, e.g., a batch-type receptacle. In some embodiments, the chlorine dioxide source produces chlorine dioxide (e.g., an apparatus such as a chlorine dioxide generator). For example, in some embodiments, the gas source comprises or is configured such that it is attached to a chlorine dioxide generator, e.g. as disclosed and claimed in U.S. Pat. No. 6,468,479, to which further reference should be made. In embodiment comprising and/or utilizing a chlorine dioxide generator, the chlorine dioxide is generated either directly as a gas, or as an aqueous (or other suitable liquid carrier) chlorine dioxide mixture/solution. The generator may be run using an excess of sodium chlorite to reduce the possibility of generating chlorine gas as an impurity. Other generally accepted methods and devices for generating chlorine dioxide which may be utilized in, and/or comprised by the present inventive methods and devices can be found in, for example, U.S. Patent No. 7,678,388, U.S. Patent No. 5,290,524, and U.S. Patent No. 5,234,678, to which further reference should be made.

Where the chlorine dioxide source comprises a solution which comprises, e.g., dissolved chlorine dioxide, the chlorine dioxide source may comprise, and methods of the invention may comprise or otherwise utilize a chlorine dioxide stripper, which is an apparatus (e.g., a countercurrent stripper, spray stripper, etc.), that uses, e.g., air to carry chlorine dioxide out of solution, and ultimately to the gas source (e.g., via a chlorine dioxide inlet).

In various embodiments of the invention, the gas source is configured to intake chlorine dioxide from a chlorine dioxide source via a chlorine dioxide inlet. The chlorine dioxide inlet may be, e.g., a part of the gas source (e.g., such that the gas source comprises the chlorine dioxide inlet), or it may be physically separate from the gas source. The chlorine dioxide inlet is configured for intake of a gaseous mixture (directly or indirectly) into the gas source from (directly or indirectly) the source of chlorine dioxide.

In one embodiment, the gas source is a nozzle which is attached, either directly or indirectly, to a chlorine dioxide source (e.g., a chlorine dioxide generator or a receptacle comprising chlorine dioxide gas or chlorine dioxide in solution). The nozzle may be configured such that it receives chlorine dioxide through a chlorine dioxide inlet. In some embodiments where the chlorine dioxide source comprises a chlorine dioxide solution, the solution travels to a chlorine dioxide stripper, which carries chlorine dioxide gas in air from the, e.g., aqueous solution, and the gaseous mixture ultimately subsequently travels to the gas source to be ejected at a target.

The inventive method comprises ejecting a gas stream of the gaseous mixture from a gas source at a velocity of 45.7 to 274.3 m/s (150 to 900 ft/sec), and contacting the gas stream with a target. For example, in some embodiments, the gas stream is ejected from the gas source at a velocity of 45.7, 53.3, 61.0, 68.6, 76.2, 83.8, 91.4, 99.1, 106.7, 114.3, 121.9, 129.5, 137.1, 144.8, 152.4, 160.0, 167.6, 175.3, 182.9, 190.5, 198.1, 205.7, 213.4, 221.0, 228.6, 236.2, 243.8, 251.5, 259.1, 266.7, or 274.3 m/s (150, 175, 200, 225, 250, 275, 300, 325, 350, 375, 400, 425, 450, 475, 500, 525, 550, 575, 600, 625, 650, 675, 700, 725, 750, 775, 800, 825, 850, 875, or 900 ft/sec), including any and all ranges and subranges therein (e.g., 45.7 to 274.3 m/s, 91.4 to 259.1 m/s, 182.9 to 243.8 m/s (150 to 900 ft/sec, 300 to 850 ft/sec, 600 to 800 ft/sec), etc.).

Persons having ordinary skill in the art will readily recognize manners of increasing and decreasing the velocity of gas ejected from the gas source, all of which may be utilized in the present invention. For example, where the gas source is a nozzle, the velocity may be increased, for example, by decreasing the diameter of the nozzle, and the velocity may be decreased, e.g., by increasing the diameter of the nozzle. Similarly, velocity may be increased or decreased by, e.g., altering a gas flow rate to and/or through the gas source.

It has been found that methods of the invention employing the aforementioned velocity (45.7 to 274.3 m/s (150 to 900 ft/sec)) of ejected gas advantageously operate to oxidize, sanitize, disinfect, and/or sterilize a target. For example, in various embodiments of the invention, the methods provided allow for advantageous oxidizing, sanitizing, disinfecting, and/or sterilizing using lower chlorine dioxide concentration-time (CT) values than prior art methods and devices.

As used herein, concentration-time ("CT"), or total concentration, equals the time-weighted average of chlorine dioxide concentration in parts per million by volume (ppmᵥ) multiplied by the exposure time in hours. In a plot of chlorine dioxide concentration versus exposure time in hours, the CT would equal the area under the curve. For example, if the time weighted average chlorine dioxide concentration over a 12-hour exposure period were 750 ppmᵥ, the CT would be 9,000 ppmv-hrs (the CT required by, for example, current EPA guidelines for applications of gaseous chlorine dioxide for building remediation). Similarly, if the time weighted average chlorine dioxide concentration over a 3-hour exposure period were 3,000 ppmᵥ, the CT would still be 9,000 ppmv-hrs. If the time weighted average chlorine dioxide concentration over a 1 minute exposure period were 3,000 ppmᵥ, the CT would be 50 ppmv-hrs.

In a gas or vapor phase application of chlorine dioxide for building remediation, typical chlorine dioxide concentrations are in the range of 500 to 3000 ppmᵥ, and exposure times are typically about 3 to 12 hours. For example, a time averaged chlorine dioxide gas concentration in the range of about 500 to 1500 ppmᵥ over a 12 hour period has been found effective for killing mold spores and eliminating allergenic effects (CT = 6000 - 18000 ppmᵥ -hrs). Similarly, a CT of 9000 ppmv-hrs has been found effective for sterilizing anthrax spores.

Any embodiment of the invention that meets the limitations requiring ejecting a gas stream of a gaseous mixture comprising 50 to 30,000 ppmᵥ chlorine dioxide from a gas source at a velocity of 45.7 to 274.3 m/s (150 to 900 ft/sec); and contacting the gas stream with an intended target falls within the scope of the present invention, regardless of the CT for any given treatment/application. However, in various embodiments of the present invention, the desired oxidation, sanitization, disinfection, and/or sterilization is achieved with a CT of 0.15 to 5,000 ppmv-hrs.

For example, in some embodiments, the desired oxidation, sanitization, disinfection, and/or sterilization is achieved with a with a CT of 0.15, 0.5, 1, 5, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950, 1,000, 1,250 , 1,500, 1,750, 2,000, 2,250 , 2,500, 2,750, 3,000, 3,250 , 3,500, 3,750, 4,000, 4,250 , 4,500, 4,750, or 5,000 ppmv-hrs, including any and all ranges and subranges therein (e.g., 1 to 4,000 ppmv-hrs, 10 to 3,500 ppmv-hrs, 15 to 3,000 ppmv-hrs, 20 to 2,500 ppmv-hrs, 25 to 2,000 ppmv-hrs, 30 to 1,500 ppmv-hrs, 30 to 500 ppmv-hrs, etc.).

Persons having ordinary skill in the art will understand that because CT is, by its nature, a function of concentration and exposure time, the CT for any given application is determined based on both of these variables. Accordingly, the same CT may be obtained using a gaseous mixture comprising a higher concentration of chlorine dioxide using a shorter exposure period, as can be obtained using a gaseous mixture comprising a lower concentration of chlorine dioxide over a longer exposure period.

In some non-limiting embodiments of the invention, the inventive methods comprise ejecting, a dose (e.g., a focused dose) of 4 to 4.5 x 10⁶ CTv, where CTv is equal to CT (in ppmv-hrs) multiplied by the velocity of the gaseous mixture ejected from the gas source (in ft/sec). For example, in some embodiments, a dose of 4, 4.5, 50, 100, 200, 300, 400, 500, 600, 700, 800, 900, 1,000, 2,000, 3,000, 4,000, 5,000, 6,000, 7,000, 8,000, 9,000, 10,000, 20,000, 30,000, 40,000, 50,000, 60,000, 70,000, 80,000, 90,000, 100,000, 110,000, 120,000, 130,000, 140,000, 150,000, 160,000, 170,000, 180,000, 190,000, 200,000, 210,000, 220,000, 230,000, 240,000, 250,000, 300,000, 400,000, 500,000, 600,000, 700,000, 800,000, 900,000, 1,000,000, 1,250,000, 1,500,000, 1,750,000, 2,000,000, 2,250,000, 2,500,000, 2,750,000, 3,000,000, 3,250,000, 3,500,000, 3,750,000, 4,000,000, 4,250,000, or 4,500,000, including any and all ranges and subranges therein (e.g., 4 to 1,000,000 CTv, 500 to 250,000 CTv, 600 to 220,000 CTv, etc.) In some embodiments of the invention, the ejected gaseous mixture contacts the target at 4 to 4.5 x 10⁶ CTv.

In some embodiments of the inventions, methods are carried out for use in application zones that have not received any pre-treatments or conditioning. In some embodiments of the invention, methods may be carried for use in an application zone that has been humidified and/or climatized, e.g., prior to or during application. For example, in some embodiments, application zones may be humidified, to, for example, relative humidity (RH) in the range of 5% to 80% (e.g., 5%, 10%, 15%, 20%, 25%, 30%, 35%, 40%, 45%, 50%, 55%, 60%, 65%, 70%, 75%, or 80%, including any and all ranges and subranges therein, e.g., 5-55%, 35-55%, 40-55%, 45-50%, 45-48%, etc.). In some embodiments, application zones may be climatized to, for example, 10 °C to about 79.4 °C (50 °F to about 175 °F) (e.g., 10.0, 12.8, 15.6, 18.3, 21.1, 23.9, 26.7, 29.4, 32.2, 35.0, 37.8, 40.6, 43.3, 46.1, 48.9, 51.7, 54.4, 57.2, 60.0, 62.8, 65.6, 68.3, 71.1, 73.9, 76.7, or 79.4 °C (50, 55, 60, 65, 70, 75, 80, 85, 90, 95, 100, 105, 110, 115, 120, 125, 130, 135, 140, 145, 150, 155, 160, 165, 170, or 175 °F), including any and all ranges and subranges therein, e.g., 15.6-32.2 °C, 18.3-29.4 °C (60-90 °F, 65-85 °F), etc.).

In embodiments of the invention, the target may be positioned/located any desired distance from the gas source. In some embodiments, the gas source is positioned 0.5 to 50 cm from the target during ejection of the gas stream, such that gas in the gas stream travels 0.5 to 50 cm from the source before contacting the target. For example, in some embodiments, the gas source is positioned 0.5, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 cm from the target, including any and all ranges and subranges therein (e.g., 0.5 to 25 cm, 1 to 10 cm, 1 to 4 cm, etc.)

The velocity of the gas as it hits the target will vary as a function of, e.g., gas flow rate to the gas source, velocity of gas ejected from the gas source, and distance of the target from the gas source. In some non-limiting embodiments, the gas stream contacts the target at a velocity of 4.6 to 152.4 m/s (15 to 500 ft/sec), e.g., 4.6, 9.1, 13.7, 15.2, 22.9, 30.5, 38.1, 45.7, 53.3, 61.0, 68.6, 76.2, 83.8, 91.4, 99.1, 106.7, 114.3, 121.9, 129.5, 137.1, 144.8, 152.4 m/s (15, 30, 45, 50, 75, 100, 125, 150, 175, 200, 225, 250, 275, 300, 325, 350, 375, 400, 425, 450, 475, or 500 ft/sec), including any and all ranges and subranges therein (e.g., 7.62 to 121.9 m/s, 12.2 to 91.4 m/s, 15.2 to 76.2 m/s (25 to 400 ft/sec, 40 to 300 ft/sec, 50 to 250 ft/sec), etc.).

The area of application according to the present invention (i.e., the area comprising the gas source and the target) may be referred to as the application zone. The application zone may be open (e.g., open to a surrounding room or building) or contained (meaning that the application zone is substantially or entirely separated from its surroundings, e.g., in a chamber, within a containment mechanism, under a hood, etc.).

In some embodiments of the invention, the target is contacted with the gas stream in an application zone wherein circulation of air per minute in the application zone is in excess of the normal rate of circulation of air in the application zone (for example, an application zone may be, e.g., a room, where the rate of air circulation is generally about 0.9 m/s(3 ft/min)). The excess rate of circulation may be achieved in any desirable manner, for example, by using a fan or blowers directed at the target surfaces or in the case of treating pipes, vessels, ducts or HVAC systems by increasing the velocity through the system. By ramping up/increasing the rate of circulation in an application zone such that it exceeds the normal rate of circulation in the application zone, the rate of circulating air can effectively increase the velocity of a gas stream at a target.

In some embodiments of the invention, the target is contacted with the gas stream in an application zone wherein circulation of air per minute in the application zone is at least 0.9 m/s (3 ft/sec), for example, at least 0.9 m/s, 1.5 m/s, 3.0 m/s, 4.6 m/s or 6.1 m/s (3 ft/sec, 5 ft/sec, 10 ft/sec, 15 ft/sec, or 20 ft/sec), including any and all ranges and subranges therein (e.g., 1.5 to 6.1 m/s (5 to 20 ft/sec), etc.). In some embodiments, the velocity of the gas stream at the target increases due to the circulation of air in the application zone.

In some embodiments of the invention, the application zone is maintained under a negative pressure, for example, by operation of a vacuum. The vacuum may be created by a device as described herein, or by another apparatus separate from the inventive device. In some embodiments, the vacuum retrieves spent gaseous mixture. In some embodiments, the retrieved spent gaseous mixture is recycled for one or more subsequent ejection cycles from the gas source.

In some embodiments, the application zone is a sealed area. For example, in some embodiments, the application zone is a brush sealed area, a flexibly-sealed area (e.g., the area under a fume hood, where the hood window has been pulled down), and/or an air-tight sealed area (i.e., a hermetically sealed area). In some embodiments, the method of the invention is performed in a sealed application zone under negative pressure, where the application zone comprises the gas source, the target, and a source of a vacuum.

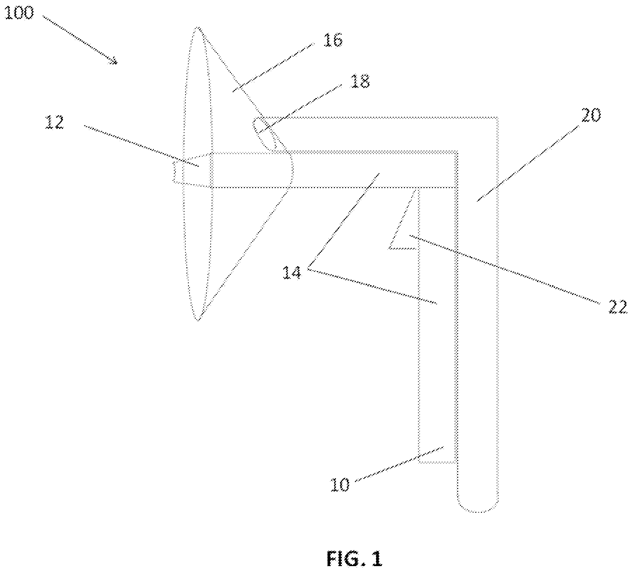

FIG. 1 is a side perspective line drawing of a device 100 for oxidizing, sanitizing, disinfecting, and/or sterilizing a target according to one example.

The device **100** of FIG. 1 comprises chlorine dioxide inlet **10**, which is configured for intake of a gaseous mixture comprising 50 to 30,000 ppmᵥ chlorine dioxide. The chlorine dioxide inlet **10** may intake the gaseous mixture from, e.g., any chlorine dioxide source (not pictured). For example, in some embodiments, chlorine dioxide inlet **10** is configured to receive/intake chlorine dioxide from a chlorine dioxide generator, e.g. as disclosed in U.S. Pat. No. 6,468,479, to which the chlorine dioxide inlet **10** may be directly or indirectly connected. Where chlorine dioxide inlet **10** intakes chlorine dioxide from a generator that generates chlorine dioxide in solution, the solution passes through a stripper en route to device **100.** In some embodiments, chlorine dioxide inlet **10** is configured to receive/intake chlorine dioxide from a chlorine dioxide source such as a receptacle comprising chlorine dioxide gas or chlorine dioxide in solution. Where the receptacle comprises a solution, the solution may have been prepared by any acceptable means (e.g., by a generator). Where the receptacle comprises chlorine dioxide in solution, the solution passes through a stripper en route to device **100.** In some embodiments, the device **100** may be attached (directly or indirectly) to a separate chlorine dioxide source, while in other embodiments, the device itself may comprise a chlorine dioxide source.

As illustrated, in the depicted embodiment, the chlorine dioxide inlet **10** is configured for intake of gaseous mixture indirectly to gas source **12** via adjoining components **14**, which may be any desired components (e.g., pipes, tubes, columns, etc.). The gas source **12** is a nozzle configured to eject a gas stream of the gaseous mixture at a velocity of 45.7 to 274.3 m/s (150 to 900 ft/sec). Chlorine dioxide inlet **10** is configured to intake the gaseous mixture from a source of chlorine dioxide (not pictured), such as, for example, a chlorine dioxide generator or receptacle.

The device **100** also comprises containment mechanism **16**, which houses gas source **12,** and is configured to define an application zone. While the containment mechanism **16** may be of any desired shape, size, and aesthetics, the containment mechanism **16** of device **100** is a clear cone with a flexible seal or brush seal or spacer (not pictured) to help provide for gas containment. Depending upon the size of containment mechanism **16** and the distance between the gas source **12** and target (not pictured), the containment mechanism **16** can serve to contain an application zone (i.e., to substantially or entirely separate the containment zone from its surroundings). As will be apparent to persons having ordinary skill in the art, in such embodiments, the size of the containment mechanism **16** (when present) can determine the concentration of chlorine dioxide in an application zone.

In some embodiments, containment mechanism **16** is configured to seal the application zone in relation to a target to be sterilized, meaning, e.g., in the depicted embodiment, that the cone of containment mechanism **16** would comprise, e.g., a seal (for example, a hermetic seal), which would come into contact with a target and/or the surroundings of a target so as to seal the target within the containment mechanism **16**, thereby creating a contained and sealed application zone which would comprise both the gas source **12** and the target.

Device **100** also comprises vacuum source **18**, which is configured to retrieve spent gaseous mixture (i.e., gaseous mixture that has been ejected from gas source **12**), by creating a vacuum within, e.g., an application zone. In the depicted embodiment, source of vacuum **18** creates a vacuum within the application zone. The vacuum sucks/draws spent gaseous mixture (and any other gas present, e.g., air within the application zone) into gas return component(s) **20**, such that the gas may be recycled, diverted, and/or disposed of elsewhere. Gas return component **20** may be any desired or acceptable component, including, but not limited to, one or more pipes, tubes, etc.

In some embodiments of the method of the invention, a device is configured to recycle the spent gaseous mixture and/or the chlorine dioxide of the spent gaseous mixture for one or more subsequent ejection cycles from the gas source. For example, in the case of device **100**, gas return component **20** may comprise, or may be connected (directly or indirectly) to a chlorine dioxide scrubber. In some embodiments, gas return component **20** is connected to a chlorine dioxide generator that comprises a chlorine dioxide scrubber. When methods of the invention comprise, and/or are connected to and/or utilize a chlorine dioxide scrubber, the scrubber is capable of removing chlorine dioxide from a mixture (e.g., an effluent or gaseous mixture) that passes through the scrubber. Any acceptable scrubber that is capable of removing chlorine dioxide from a mixture may be used. For example, in some embodiments, a scrubber may comprise activated carbon, an alkaline solution (e.g., ascorbic acid, hydrogen peroxide, sodium sulfite, etc.), water, etc. In some embodiments, the method comprises, and/or is connected to and/or utilizes one or more scrubbers that are configured to remove other constituents from a mixture. In some embodiments, devices comprise and/or are connected to scrubbers, which may in turn be connected to, e.g., the chlorine dioxide source, such that scrubbed chlorine dioxide may be returned to the chlorine dioxide source where it may be recycled for use in subsequent ejection cycles.

When gaseous mixture (often with air) is returned to a scrubber (e.g., a scrubber comprised by the invention, a separate scrubber, and/or a scrubber comprised by a chlorine dioxide source such as a generator), the chlorine dioxide can be scrubbed from the mix for recycle in future ejection cycles.

In some embodiments, gas return component **20** comprises, or is attached to a filter (e.g., a HEPA filter), which the spent gas is passed through before it is ultimately, e.g., recycled, diverted, and/or disposed of.

Device **100** also comprises trigger **22**, which may be configured to, for example, initiate and/or terminate ejection periods/cycles, and/or to control the velocity at which the gaseous mixture is ejected.

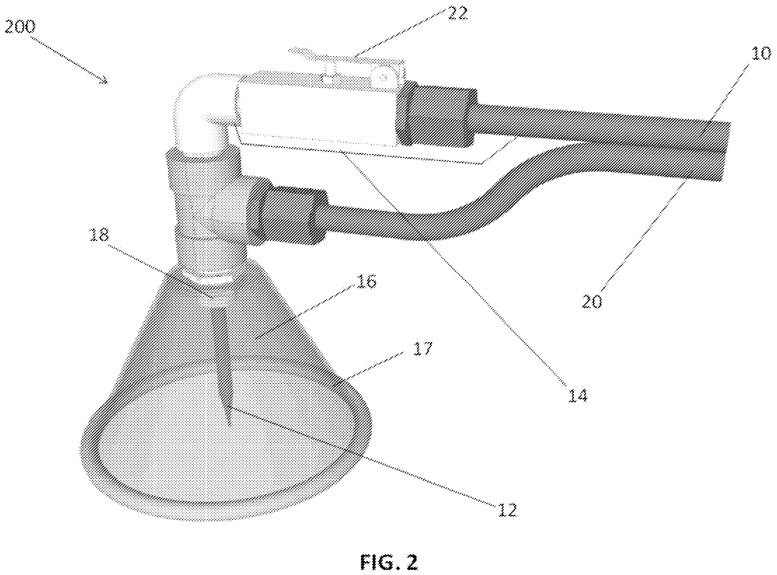

FIG. 2 illustrates a device **200** for oxidizing, sanitizing, disinfecting, and/or sterilizing a target.

The device **200** of FIG. 2 comprises chlorine dioxide inlet **10**, which is configured for intake of a gaseous mixture comprising 50 to 30,000 ppmᵥ chlorine dioxide, and which is connected to, and retrieves chlorine dioxide from a solid-state chlorine dioxide generator for generating an aqueous solution of chlorine dioxide (not pictured). Gas is obtained when chlorine dioxide solution from the generator is fed through a stripper, which may be a part of the generator, or a part of the device of the invention, or a separate apparatus that may be connected (directly or indirectly) to the generator and/or to the device of the invention. In the depicted embodiment, the stripper is connected to the chlorine dioxide generator and to the device **200**, such that solution from the generator passes through the stripper, and subsequently toward and through chlorine dioxide inlet **10.** In one embodiment, chlorine dioxide solution supply to the gas stripper uses 0.78 grams per minute of ClO₂ or about a 60% strip efficiency, and 1.3 grams total feed at 3 g/L would equal about 450 mL per minute of feed solution. Accordingly, in some embodiments, 0.78 grams per minute are needed, but strip efficiency is less than 100% (e.g., 60%), so additional ClO₂ is fed (e.g., 1.3 grams per minute). In some of such embodiments, a solution, prior to entering the stripper, may comprise, e.g., 3 grams ClO₂ per liter, and in some embodiments, about 435 ml per minute of solution may be used.

The chlorine dioxide inlet **10** of device **200** is configured for intake of gaseous mixture indirectly to gas source **12**, which is a nozzle, via adjoining components **14.**

The device **200** also comprises containment mechanism **16**, which is a clear cone comprising flexible seal **17**, which is configured to establish a hermetic seal. Containment mechanism **16** houses gas source **12**, and is configured to define an optionally contained and sealed application zone which may comprise the gas source **12** and a target (not pictured). The device **200** comprises vacuum source **18**, which sucks/draws spent gaseous mixture and optionally air from the application zone into gas return component **20**, which is a tube. While vacuum source **18** sucks and/or draws gas, the actual perpetuator or provider of the vacuum (e.g., a fan, air pump, etc.) may be located elsewhere within or outside of the device. In the depicted embodiment, vacuum source **18** is connected to gas return component **20**, through which spent gaseous mixture is vacuumed as it leaves the application zone. The actual perpetuator/provider of the vacuum may be any acceptable means (e.g., a fan, air pump, etc.) that may be a part of, or separate from, but connected to (including connectable to), the device.

Trigger **22** of device **200** is configured to start and stop ejection cycles of the gaseous mixture.

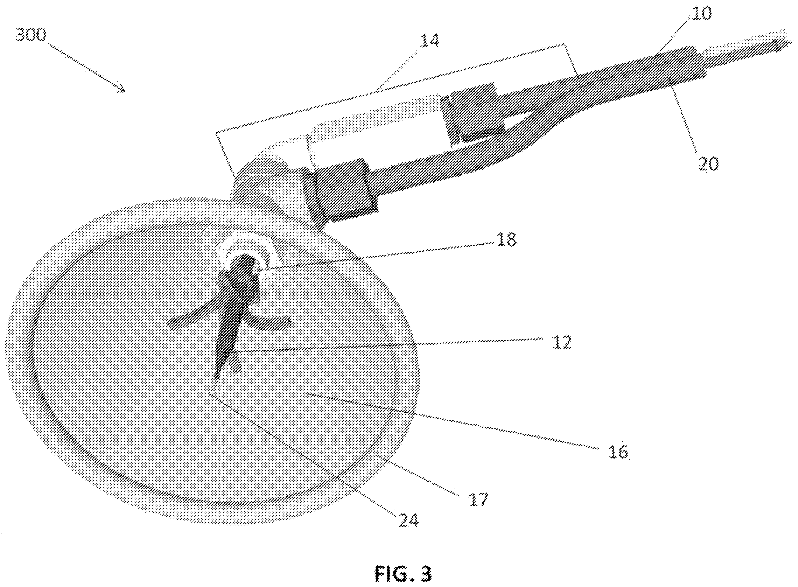

FIG. 3 illustrates a device **300** for oxidizing, sanitizing, disinfecting, and/or sterilizing a target.

The device **300** of FIG. 3 comprises chlorine dioxide inlet **10**, which is configured for intake of a gaseous mixture comprising 50 to 30,000 ppmᵥ chlorine dioxide, and which is connected to, and retrieves chlorine dioxide from a batch-type chlorine dioxide source (not pictured). In particular, the depicted device **300** comprises, or may be connected to a receptacle (e.g., a 0.5-5 liter receptacle) comprising chlorine dioxide gas or chlorine dioxide solution. Where the chlorine dioxide source for device **300** is a receptacle comprising chlorine dioxide solution, the device also utilizes a chlorine dioxide stripper, which may be a part of the receptacle, or a part of the device **300**, or a separate apparatus that may be connected (directly or indirectly) to the receptacle and device **300.** In the depicted embodiment, the stripper is a separate apparatus connected to the chlorine dioxide solution receptacle and to the device **300**, such that solution from the receptacle passes to the stripper, where the chlorine dioxide gas is carried out of solution with air in a gaseous mixture comprising 50 to 30,000 ppmᵥ chlorine dioxide. The gaseous mixture subsequently travels toward and through chlorine dioxide inlet **10**, and ultimately leaves the device **300** via gas source **12** as gaseous mixture **24.**

### EXAMPLES

The bio gun device of FIG. 3 was configured such that a gaseous mixture of 3,000 ppmᵥ chlorine dioxide gas in air was fed through the chlorine dioxide inlet at a gas flow rate of 10 liters per minute. During ejection, the gaseous mixture exited the device from gas source, which was the gun nozzle, and was ejected from the nozzle, which had a 0.10 cm diameter, at 213.4 m/s (700 feet/sec). For the tests, the nozzle was placed 2.54 cm from the target, which was a *Bacillus atrophaeus* spore strip manufactured by SGM Biotech, having a titer of 10⁶ *Bacillus atrophaeus.*

Ejection cycles were run using the preceding setup, for 30 second, 1 minute, 2 minute, and 5 minute exposure times in contained application zones that received no pre-humidification treatment prior to the testing, and that were pre-humidified for one hour at about 23.9 °C (75 degrees Fahrenheit) (i.e., 23.9 °C ± 2.8 °C (75 °F ± 5°F). Following application of the gaseous mixture to each target for the indicated exposure time, the targets were evaluated and were tested to determine whether sterilization had been successful (i.e., whether there was at least a 6-log reduction of *Bacillus atrophaeus,* thereby indicating statistical destruction of the bacterial population). While every test resulted in oxidation of the target, the results from the sterilization testing are provided below in Tables I and II. In the tables, CT is presented in ppmv-hrs, "+" indicates less than a 6-log reduction of *Bacillus atrophaeus,* and "-" indicates at least a 6-log reduction of *Bacillus atrophaeus.*

**Table I**

| **Group 1 Spore Strips (Pre-Humidified for One Hour at about 23.9 °C (75 degrees F))** | | | | |
|---|---|---|---|---|
| **Sample Rep. #** | **Exposure Time** | | | |
| | **30 Seconds** (CT = 25) | **1 Minute** (CT = 50) | **2 Minutes** (CT = 100) | **5 Minutes** (CT = 250) |
| 1 | + | - | - | - |
| 2 | - | - | - | - |
| 3 | - | - | - | - |
| 4 | - | | | |

**Table II**

| **Group 2 Spore Strips (No Pre-Humidification Prior to Test)** | | | | |
|---|---|---|---|---|
| **Sample Rep. #** | **Exposure Time** | | | |
| | **30 Seconds** (CT = 25) | **1 Minute** (CT = 50) | **2 Minutes** (CT = 100) | **5 Minutes** (CT = 250) |
| 1 | + | + | + | - |
| 2 | + | + | + | - |
| 3 | + | - | - | - |
| 4 | - | | | |

As demonstrated by the foregoing results, where application zones were pre-humidified to > 23.9 °C (75 degrees Fahrenheit), methods of the invention achieved successful sterilization of the targets for all applications using exposure times greater than or equal to 1 minute, and having a CT of greater than or equal to 50. 75% of applications to targets for 30 seconds at a CT of 25 resulted in successful sterilization. Where application zones did not receive any pre-humidification treatment, methods of the invention achieved successful sterilization of all targets for exposure times of at least 5 minutes at a CT of 250, and some sterilization was realized for exposure times of 30 seconds, 1 minute, and 2 minutes, at CTs of 25, 50, and 100, respectively. These results demonstrate the ability of the present invention to advantageously oxidize, sanitize, disinfect, and/or sterilize targets at considerably lower CTs than methods of the prior art.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has" and "having"), "include" (and any form of include, such as "includes" and "including"), and "contain" (and any form contain, such as "contains" and "containing") are open-ended linking verbs. As a result, a method or device that "comprises", "has", "includes" or "contains" one or more steps or elements possesses those one or more steps or elements, but is not limited to possessing only those one or more steps or elements. Likewise, a step of a method that "comprises", "has", "includes" or "contains" one or more features possesses those one or more features, but is not limited to possessing only those one or more features.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of' and "consisting essentially of".

The phrase "consisting essentially of' or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, or method.

Where one or more ranges are referred to throughout this specification, each range is intended to be a shorthand format for presenting information, where the range is understood to encompass each discrete point within the range as if the same were fully set forth herein.

While several aspects and embodiments of the present invention have been described and depicted herein, alternative aspects and embodiments may be affected by those skilled in the art to accomplish the same objectives. Accordingly, this disclosure and the appended claims are intended to cover all such further and alternative aspects and embodiments as fall within the scope of the invention.

## Claims

1. A method of oxidizing, sanitizing, disinfecting, and/or sterilizing a target comprising:
ejecting a gas stream of a gaseous mixture comprising 50 to 30,000 ppmᵥ chlorine dioxide from a gas source at a velocity of 45.7 m/s to 274.3 m/s (150 ft/sec to 900 ft/sec); and
contacting the gas stream with the target.

2. The method according to claim 1, wherein the contacting is for a concentration-time (CT) of 25 to 2,000 ppmv-hours.

3. The method according to claim 1 or 2, wherein the gaseous mixture comprises 2,000 to 10,000 ppmᵥ chlorine dioxide.

4. The method according to any one of claims 1 to 3, wherein the gas stream is ejected from the gas source at a velocity of 45.7 m/s to 213.4 m/s (150 ft/sec to 700 ft/sec).

5. The method according to any one of claims 1 to 3, wherein the gas stream is ejected from the gas source at a velocity of 182.9 m/s to 243.8 m/s (600 ft/sec to 800 ft/sec).

6. The method according to any one of claims 1 to 5, wherein the target is selected from:
(i) at least a portion of a ceiling or wall;
(ii) at least a portion of a medical instrument; and
(iii) at least a portion of a medical procedural area.

7. The method according to any one of claims 1 to 6, wherein the gas stream contacts the target at a velocity of 4.6 m/s to 152.4 m/s (15 ft/sec to 500 ft/sec).

8. The method according to any one of claims 1 to 6, wherein the gas stream contacts the target at a velocity of 61.0 m/s to 152.4 m/s (200 ft/sec to 500 ft/sec).

9. The method according to any one of claims 1 to 8, wherein the gas source is positioned 0.5 to 25 cm from the target during ejection of the gas stream.

10. The method according to any one of claims 1 to 9, wherein the gas source is positioned 0.5 to 10 cm from the target during ejection of the gas stream.

11. The method according to any one of claims 1 to 10, wherein the contacting is performed for an exposure time of 30 seconds to 5 minutes.

12. The method according to any one of claims 1 to 11, wherein the gas source is a nozzle.

13. The method according to any one of claims 1 to 12, wherein the contacting is performed in an application zone, wherein circulation of air per minute in the application zone is at least 1.5 m/s (5 ft/sec), and wherein the velocity of the gas stream at the target increases due to the circulation of air in the application zone.

14. The method according to any one of claims 1 to 13, wherein the contacting is performed in a contained and sealed application zone that contains the gas source and the target, wherein the application zone is maintained under a negative pressure.

15. The method according to claim 14, wherein the method further comprises applying a vacuum to the application zone and retrieving spent gaseous mixture for recycling of the spent gaseous mixture and/or the remaining chlorine dioxide of the spent gaseous mixture for one or more subsequent ejection cycles from the gas source.

## Patentansprüche

1. Verfahren zum Oxidieren, Säubern, Desinfizieren und/oder Sterilisieren eines Ziels, umfassend:
Ausstoßen eines Gasstroms eines gasförmigen Gemischs, das 50 bis 30.000 ppmᵥ Chlordioxid umfasst, von einer Gasquelle mit einer Geschwindigkeit von 45,7 m/s bis 274,3 m/s (150 Fuß/Sek. bis 900 Fuß/Sek.); und
Inkontaktbringen des Gasstroms mit dem Ziel.

2. Verfahren nach Anspruch 1, wobei das Inkontaktbringen über eine Konzentrationszeit (CT) von 25 bis 2.000 ppmᵥ-Stunden erfolgt.

3. Verfahren nach Anspruch 1 oder 2, wobei das gasförmige Gemisch 2.000 bis 10.000 ppmᵥ Chlordioxid umfasst.

4. Verfahren nach einem der Ansprüche 1 bis 3, wobei der Gasstrom von der Gasquelle mit einer Geschwindigkeit von 45,7 m/s bis 213,4 m/s (150 Fuß/Sek. bis 700 Fuß/Sek.) ausgestoßen wird.

5. Verfahren nach einem der Ansprüche 1 bis 3, wobei der Gasstrom von der Gasquelle mit einer Geschwindigkeit von 182,9 m/s bis 243,8 m/s (600 Fuß/Sek. bis 800 Fuß/Sek.) ausgestoßen wird.

6. Verfahren nach einem der Ansprüche 1 bis 5, wobei das Ziel ausgewählt ist aus:
(i) mindestens einem Teil einer Decke oder einer Wand;
(ii) mindestens einem Teil eines medizinischen Instruments; und
(iii) mindestens einem Teil eines Bereichs für ein medizinisches Verfahren.

7. Verfahren nach einem der Ansprüche 1 bis 6, wobei der Gasstrom mit einer Geschwindigkeit von 4,6 m/s bis 152,4 m/s (15 Fuß/Sek. bis 500 Fuß/Sek.) in Kontakt mit dem Ziel gelangt.

8. Verfahren nach einem der Ansprüche 1 bis 6, wobei der Gasstrom mit einer Geschwindigkeit von 61,0 m/s bis 152,4 m/s (200 Fuß/Sek. bis 500 Fuß/Sek.) in Kontakt mit dem Ziel gelangt.

9. Verfahren nach einem der Ansprüche 1 bis 8, wobei die Gasquelle während dem Ausstoßen des Gasstroms 0,5 bis 25 cm von dem Ziel entfernt positioniert ist.

10. Verfahren nach einem der Ansprüche 1 bis 9, wobei die Gasquelle während dem Ausstoßen des Gasstroms 0,5 bis 10 cm von dem Ziel entfernt positioniert ist.

11. Verfahren nach einem der Ansprüche 1 bis 10, wobei das Inkontaktbringen über eine Expositionszeit von 30 Sekunden bis 5 Minuten ausgeführt wird.

12. Verfahren nach einem der Ansprüche 1 bis 11, wobei die Gasquelle eine Düse ist.

13. Verfahren nach einem der Ansprüche 1 bis 12, wobei das Inkontaktbringen in einer Applikationszone ausgeführt wird, wobei die Luftzirkulation pro Minute in der Applikationszone mindestens 1,5 m/s (5 Fuß/Sek.) beträgt, und wobei die Geschwindigkeit des Gasstroms an dem Ziel durch die Luftzirkulation in der Applikationszone zunimmt.

14. Verfahren nach einem der Ansprüche 1 bis 13, wobei das Inkontaktbringen in einer abgeschlossenen und abgedichteten Applikationszone ausgeführt wird, welche die Gasquelle und das Ziel enthält, wobei die Applikationszone unter Unterdruck gehalten wird.

15. Verfahren nach Anspruch 14, wobei das Verfahren ferner das Anwenden eines Vakuums in der Applikationszone umfasst sowie das Rückgewinnen des verbrauchten Gasgemischs zur Wiederverwendung des verbrauchten Gasgemischs und/oder des verbliebenen Chlordioxids des verbrauchten Gasgemischs für einen oder mehrere folgende Ausstoßzyklen von der Gasquelle.

## Revendications

1. Procédé d'oxydation, d'assainissement, de désinfection et/ou de stérilisation d'une cible comprenant :
l'éjection d'un courant de gaz d'un mélange gazeux comprenant de 50 à 30 000 ppmᵥ de dioxyde de chlore provenant d'une source de gaz à une vitesse de 45,7 m/s à 274,3 m/s (150 pieds/s à 900 pieds/s) ; et
mise en contact du courant de gaz avec la cible.

2. Procédé selon la revendication 1, le contact étant pour un temps de concentration (CT) compris entre 25 à 2 000 ppmᵥ-heure.

3. Procédé selon la revendication 1 ou 2, le mélange gazeux comprenant de 2 000 à 10 000 ppmᵥ de dioxyde de chlore.

4. Procédé selon l'une quelconque des revendications 1 à 3, le courant de gaz étant éjecté de la source de gaz à une vitesse comprise entre 45,7 m/s et 213,4 m/s (150 pieds/s à 700 pieds/s).

5. Procédé selon l'une quelconque des revendications 1 à 3, le courant de gaz étant éjecté de la source de gaz à une vitesse comprise entre 182,9 m/s et 243,8 m/s (600 pieds/s à 800 pieds/s).

6. Procédé selon l'une quelconque des revendications 1 à 5, ladite cible étant choisie parmi :
(i) au moins une partie d'un plafond ou d'un mur ;
(ii) au moins une partie d'un instrument médical ; et
(iii) au moins une partie d'une zone de procédure médicale.

7. Procédé selon l'une quelconque des revendications 1 à 6, le courant de gaz entrant en contact avec la cible à une vitesse comprise entre 4,6 m/s et 152,4 m/s (15 pieds/s à 500 pieds/s).

8. Procédé selon l'une quelconque des revendications 1 à 6, le courant de gaz entrant en contact avec la cible à une vitesse comprise entre 61,0 m/s et 152,4 m/s (200 pieds/s à 500 pieds/s).

9. Procédé selon l'une quelconque des revendications 1 à 8, la source de gaz étant positionnée de 0,5 à 25 cm de la cible pendant l'éjection du courant de gaz.

10. Procédé selon l'une quelconque des revendications 1 à 9, la source de gaz étant positionnée de 0,5 à 10 cm de la cible pendant l'éjection du courant de gaz.

11. Procédé selon l'une quelconque des revendications 1 à 10, la mise en contact étant effectuée pendant un temps d'exposition compris entre 30 secondes et 5 minutes.

12. Procédé selon l'une quelconque des revendications 1 à 11, la source de gaz étant une buse.

13. Procédé selon l'une quelconque des revendications 1 à 12, la mise en contact étant effectuée dans une zone d'application, la circulation de l'air par minute dans la zone d'application étant d'au moins 1,5 m/s (5 pieds/s), et la vitesse du courant de gaz au niveau de la cible augmentant en raison de la circulation de l'air dans la zone d'application.

14. Procédé selon l'une quelconque des revendications 1 à 13, la mise en contact étant effectuée dans une zone d'application contenue et scellée qui contient la source de gaz et la cible, la zone d'application étant maintenue sous une pression négative.

15. Procédé selon la revendication 14, le procédé comprenant en outre l'application d'un vide à la zone d'application et la récupération du mélange gazeux usé pour le recyclage du mélange gazeux usé et/ou du dioxyde de chlore restant du mélange gazeux usé pour au moins un cycle d'éjection ultérieur à partir de la source de gaz.
